# NEUE EUROPÄISCHE PATENTSCHRIFT

(11) **EP 0 369 043 B2**
(45) Veröffentlichungstag und Bekanntmachung des Hinweises auf die Entscheidung über den Einspruch: **31.07.1996**
(45) Hinweis auf die Patenterteilung: 10.02.1993
(21) Anmeldenummer: 88118978.1
(22) Anmeldetag: 14.11.1988
(51) Int. Cl.: A61C 1/18

(54) **Zahnärztliches Handstück mit Mitteln zum kompatiblen Anschluss an unterschiedlich gestaltete Drehkupplungen**
Dental handpiece with means providing a compatible connection to different types of rotational joints
Pièce à main dentaire pourvue de moyens qui permettent une connexion compatible avec divers types d'accouplement rotatif

(43) Veröffentlichungstag der Anmeldung: 23.05.1990
(73) Patentinhaber: SIEMENS AKTIENGESELLSCHAFT, 80333 München (DE)
(72) Erfinder: Fleer, Ernst-Otto, Ing. grad., D-6140 Bensheim 3 (DE)

(56) Entgegenhaltungen:
- WO-A-85/00281
- DE-A- 3 215 255
- US-A- 4 353 697
- US-A- 4 403 959
- Prospekt der Dentalwerke Bürmoos Nr.624/78

## Beschreibung

Die Erfindung bezieht sich auf eine zahnärztliche Handstückanordnung nach dem Oberbegriff des Patentanspruches 1.

Ein solches Handstück ist beispielsweise aus DE-A1 3 215 255 bekannt. Die in diesem Dokument offenbarte Handstückanordnung ist nach dem Baukastenprinzip aufgebaut und kann aus mehreren, unterschiedlich gestalteten, wahlweise miteinander kombinierbaren Bauteilen bzw. Baugruppen zusammengesetzt sein. Die Bauteile bzw. Baugruppen beziehen sich nicht nur auf das Kopfteil und die Griffhülse (dort mit werkzeug- und antriebsseitigem Griffhülsenteil bezeichnet), sondern auch auf die Versorgungsteile, an denen die Versorgungsschläuche angeschlossen sind. Durch Zusammensetzen der unterschiedlich gestalteten Bauteile bzw. Baugruppen soll es möglich sein, Handstücke mit unterschiedlichen Antrieben und unterschiedlichen Anschlüssen zu konfektionieren bzw. durch einen schnellen Wechsel der Bauteile eine optimale Zusammenstellung des Handstückes zu erzielen, um z.B. das gleiche Handstück alternativ mit einem luft- oder elektromotorischen Antrieb betreiben zu können.

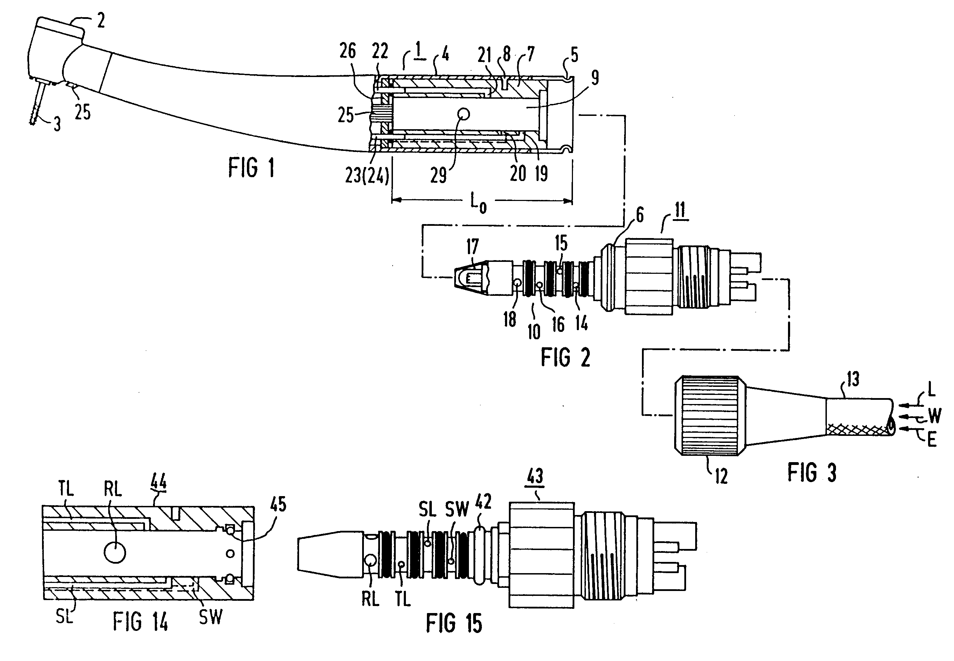

Das Dokument zeigt in einer Ausführungsform (Fig. 1) auf, wie man drei unterschiedlich gestaltete Kopfteile, drei unterschiedlich gestaltete Griffhülsenteile mit drei unterschiedlich gestalteten Versorgungsteilen kombinieren kann. Von den drei Versorgungsteilen ist eines als Drehkupplung ausgebildet und diese in mehreren Varianten beschrieben. Die anderen beiden Versorgungsteile sind nicht als Drehkupplungen ausgebildet. Das eine der beiden beinhaltet vielmehr einen Schraubanschluß für eine drehfeste Verbindung des Griffhülsenteils mit dem Versorgungsschlauch, das andere beinhaltet einen integrierten Motor. Bei diesem Teil handelt es sich also um ein Versorgungsteil mit Antrieb und nicht um ein Kupplungsteil, mit dem lediglich diverse Medien vom Versorgungsschlauch zum Handstück übertragen werden sollen.

Aus der DE-PS 25 49 177 (US-PS 40 80 737) ist eine Kupplungsvorrichtung bekannt, die in einer Ausführungsform als Drehkupplung ausgebildet ist. An diese Drehkupplung können verschiedene Handstücke betriebmäßig, d.h. ohne Zuhilfenahme von Werkzeugen, an- und abgekuppelt werden. An der Kupplungsstelle werden außer den Medien Luft und Wasser auch noch Spannung bzw. Licht angeboten. Zur Übertragung von Licht sind im Zentrum der Drehkupplung ein lichtzuführendes Element in Form eines Lichtleiters und an der Stirnseite des Handstückes ein lichtweiterführendes Element in Form eines weiteren Lichtleiters angeordnet. Im gekuppelten Zustand von Handstück und Drehkupplung stehen sich die Enden der beiden Lichtleiter korrespondierend gegenüber.

In der DE-34 31 052 (US-PS 46 55 709) ist eine Kupplungsvorrichtung beschrieben, die eine Möglichkeit aufzeigt, Turbinenhandstücke an eine Drehkupplung anschließen zu können, und zwar sowohl Turbinenhandstücke mit integrierter Lampe als auch solche mit in der Drehkupplung angeordneter Lampe. Hierzu enthält der zapfenförmige Kupplungskörper, der im gekuppelten Zustand in eine entsprechend ausgebildete Aufnahmehülse des Handstückes eingreift und an dem sowohl die Kühl- als auch die Antriebsmittel und darüber hinaus auch Spannung zur Speisung der elektrischen Lampe bereitgestellt werden, eine Schleifkontakte aufweisende Kontaktanordnung, die derart ausgebildet und angeordnet ist, daß an ihr alternativ die Kontakte einer im Kupplungskörper der Drehkupplung angeordneten Lampe oder die Anschlußkontakte einer in einem auf den Kupplungskörper aufsetzbaren Hamdstück angeordneten Lampe anschließbar sind.

Ein Vorteil dieser Kupplungsvorrichtung ist, daß an ein und dieselbe Drehkupplung Turbinenhandstücke sowohl mit integrierter Lampe als auch mit extern angeordneter Lampe alternativ anschließbar sind, wobei der Austausch von Lampe gegen eine Steckverbindung und umgekehrt vom Benutzer des Handstückes leicht selbst vorgenommen werden kann. Nachdem die Turbinenhandstücke konstruktiv auf die eine Drehkupplung abgestimmt sind, ist es demnach nach dem Stand der Technik nicht möglich, diese Handstücke an maßlich anders gestaltete Drehkupplungen anzukuppeln.

Davon ausgehend, daß auf dem Markt unterschiedliche Konstruktionen solcher Drehkupplungen bekannt sind, diese aber jeweils nur zu Handstücken eines ganz bestimmten Typs (meist des Drehkupplungsherstellers) passen, d.h. es sind nur Handstücke eines Typs kompatibel zu einer bestimmten Drehkupplung, liegt der im Anspruch 1 angegebenen Erfindung die Aufgabe zugrunde, eine Möglichkeit zu schaffen, um Handstücke unterschiedlichen Typs ohne größeren konstruktiven und Montageaufwand an unterschiedlich gestaltete Drehkupplungen anschließen zu können ohne die Medienübergabe im Handstück verändern zu müssen. Die Handstücke können Turbinenhandstücke, Handstücke mit Luft- oder Elektromotor, Spritzhandstücke oder Handstücke zur Zahnsteinentfernung sein.

Es ist möglich, für verschiedene Drehkupplungen verschiedene Einsätze vorzusehen, deren Innenkonturen und deren Leitungswege für die Übertragung der Antriebs- und Kühlmedien auf die verschiedenen Drehkupplungen passend ausgerichtet sind, deren Anschlußmaße zur Übertragung der Medien zum Handstück jedoch einheitlich auf das Handstück abgestimmt sind. Damit lassen sich nicht nur die unterschiedlichsten Handstücke mit unterschiedlichen Drehkupplungen paaren; auf diese Weise wird auch ein einheitliches Drehkupplungssystem geschaffen, ohne daß die Hersteller solcher Handstücke ihr Handstückkonzept, insbesondere in bezug auf die Lichtführung sowie die konstruktive Gestaltung der Drehkupplung, abändern müßten.

Vorteilhaft ist es, wenn die Einsätze gleichen Außendurchmesser, gleiche Länge und in bezug auf den Anschluß zum Handstück gleiches Lochbild bezüglich der Übertragung der Medien aufweisen. Bei Lichtübertragung ist es vorteilhaft, wenn, wie an sich bekannt, das Ende des das Licht weiterführenden Lichtleiters im Zentrum des Handstückes angeordnet ist und von einer ebenfalls zentrisch im Zapfen der Drehkupplung angeordneten Lampe gespeist wird. Bei der anderen denkbaren Ausführung, bei der an der Drehkupplung Spannung angeboten wird, ist es vorteilhaft, wenn im Einsatzteil eine Lampenfassung vorgesehen ist und die entsprechenden Anschlußkontakte zu Schleifkontakten führen, welche im gekuppelten Zustand mit entsprechenden Schleifringen der Drehkupplung kontaktieren. Für beide Ausführungen kann der das Licht weiterführende Lichtleiter im Handstück die gleiche Position haben, unabhängig davon, ob sich die Lampe in der Drehkupplung oder im Einsatzteil befindet; d.h. die Handstücke sind für beide Ausführungen, also für Lampe in der Drehkupplung und im Einsatzteil, absolut gleich.

Nachdem die Handstücke unterschiedlicher Hersteller in der Regel bezüglich Durchmesser, Anschluß und Rastung des Handstückes an die Drehkupplung teilweise unterschiedlich ausgeführt sind, ist es weiterhin vorteilhaft, die Anschluß- und Rastmittel für den Anschluß an die Drehkupplung den Einsatzteilen zuzuordnen.

Wenn, wie gemäß einer weiteren vorteilhaften Ausgestaltung vorgeschlagen, der hülsenförmige Endabschnitt des Handstückes, als relativ dünne Hülse ausgebildet ist, praktisch also nur einen äußeren Hüllmantel des Handstückes bildet, ansonsten das Handstück in diesem Bereich von dem Einsatzteil ausgefüllt wird, so läßt sich eine besonders gute maßliche Anpassung an die unterschiedlichen Durchmesser des Handstückes und den der anzupassenden Drehkupplung erzielen.

Weitere vorteilhafte Ausgestaltungen und Weiterbildungen der Erfindung sind aus den Unteransprüchen und der nachfolgenden Beschreibung mehrerer Ausführungsbeispiele zu entnehmen.

Es zeigen:
Figur 1 ein zahnärztliches Handstück,
Figur 2 eine erste Ausführung einer Drehkupplung,
Figur 3 ein Anschlußteil einer Versorgungsleitung,
Figur 4 das in Figur 1 gezeigte Handstück mit herausgenommenem Einsatzteil,
Figur 5 einen Schnitt entlang der Linie V/V in Figur 4,
Figuren 6 und 7 die aus den Figuren 1 und 2 entnehmbare Kombination von Einsatzteil und Drehkupplung,
Figur 8 einen Schnitt entlang der Linie VIII/VIII in Figur 6,
Figuren 9 und 10 eine zweite Kombination von Einsatzteil und Drehkupplung,
Figuren 11 und 12 eine dritte Kombination von Einsatzteil und Drehkupplung,
Figur 13 eine Frontansicht auf das Einsatzteil gemäß Figur 11,
Figuren 14, 15 eine gegenüber Figuren 6, 7 modifizierter Ausführungsform eines Einsatzteiles mit zugehöriger Drehkupplung.

Die Figur 1 zeigt, teilweise im Längsschnitt, ein allgemein mit 1 bezeichnetes Turbinenhandstück, an dessen Kopfteil 2 in bekannter Weise ein nicht dargestellter Druckluftantrieb für ein rotierendes Werkzeug 3 angeordnet ist. Im rückwärtigen Teil des Handstückes, also dem Kopfteil 2 abgewandt, bildet das Handstück über einen Längenabschnitt Lₒ eine relativ dünne, praktisch nur den äußeren Mantel des Handstückes bildende Hülse 4, welche am Ende eine federnde Einformung 5 enthält, die zusammen mit einem in Figur 2 mit 6 bezeichneten Ringbund eine Rasteinrichtung zur axialen Halterung und Fixierung der in Figur 2 gezeigten Drehkupplung bildet.

In der Hülse 4 des Handstückes 1 ist ein Einsatzteil 7 eingesetzt, welches mit Hilfe eines geeigneten Befestigungsteils 8 (Stift, Schraube, od.dgl.) gegen Verdrehung und Herausfallen gesichert ist, jedoch bei Bedarf nach Lösen des Befestigungsteiles axial aus dem Handstück entnommen werden kann. Das Einsatzteil 7 ist weitgehend rotationssymmetrisch ausgebildet und enthält eine zentrische Bohrung 9 zur Aufnahme eines Zapfens 10 der in Figur 2 mit 11 bezeichneten Drehkupplung. An der Drehkupplung 11 ist mit Hilfe einer Anschlußarmatur 12 (Figur 3) ein Versorgungsschlauch 13 anschließbar, der die Versorgungsmedien Luft (L), Wasser (W) und elektrische Energie (E) an die Drehkupplung heranführt. Am Zapfen 10 der Drehkupplung 11 sind radiale Bohrungen 14, 15, 16 für den Austritt von Spraywasser, Sprayluft und Treibluft vorgesehen. Die Bohrungen münden in Ringkanäle, welche durch nicht näher bezeichnete O-Ringe abgedichtet sind. Die elektrische Energie wird über entsprechende Leitungen zu einer stirnseitig des Zapfens 10 angeordneten elektrischen Lampe 17 geführt. Mit 18 ist eine weitere Bohrung im Zapfen 10 bezeichnet, über die Rückluft vom im Handstück angeordneten Antrieb kommend in die Drehkupplung und dort über geeignete Austrittsöffnungen ins Freie geleitet werden kann.

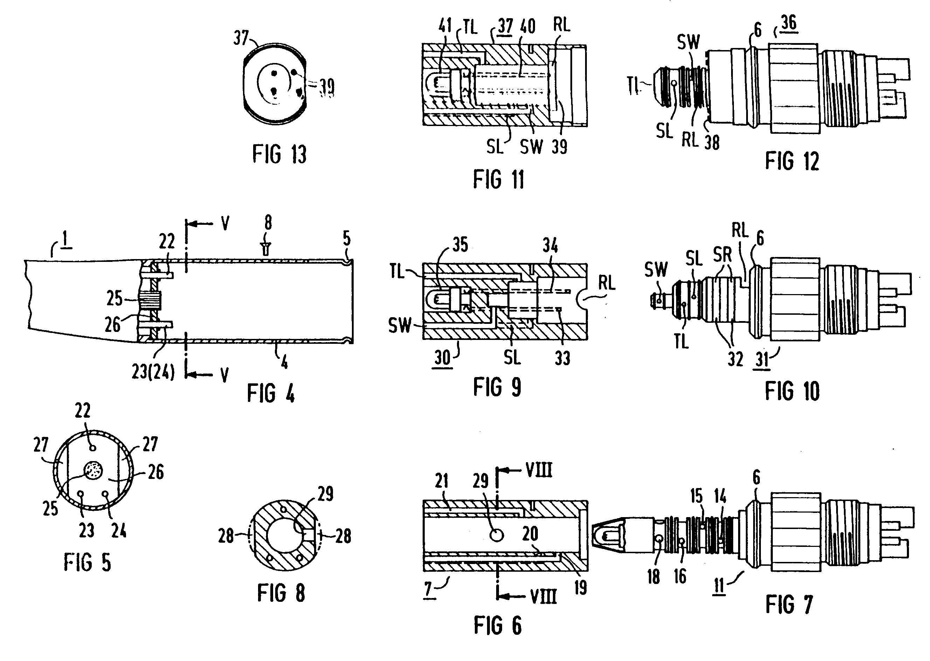

Korrespondierend zu den Bohrungen 14 bis 16 bzw. zu den Ringkanälen, in die die Bohrungen münden, sind im Einsatzteil 7 Leitungskanäle 19 bis 21 für die Übernahme der Medien Spraywasser (SW), Sprayluft (SL) und Treibluft (TL) vorgesehen. Die Leitungskanäle übernehmen die Medien vom Anschlußzapfen 10 und führen sie dann zur Stirnseite des Einsatzteiles 7. Von der Stirnseite des Einsatzteiles werden dann die Medien über Leitungen 22, 23 und 24 in bekannter Weise zum Kopfteil des Handstückes geführt. Die Enden der Leitungen 22, 23, 24 sind zusammen mit dem Ende eines Lichtleiters 25 in einer Halterung 26 gefaßt. Diese ist so ausgebildet, daß beidseitig Öffnungen 27 zur Durchführung der Rückluft (RL) vorhanden sind (Figur 5). Entsprechende Durchströmöffnungen bzw. Kanäle 28 werden auch zwischen Einsatzteil 7 und Hülse 4 durch beidseitige Abflachungen im Einsatzteil gebildet (Figuren 6 und 8). Die Rückluft kann so zwischen Außenhülse 4 des Handstückes und dem Einsatzteil 7 zurückströmen und schließlich über eine Querbohrung 29 (Figur 8) in die Bohrung 18 des Zapfens 10 geleitet werden.

Im montierten Zustand des Einsatzteiles 7 und im eingesteckten Zustand der Drehkupplung 11 sind die die einzelnen Medien führenden Abschnitte miteinander verbunden, wobei entsprechende Dichtelemente an den jeweiligen Verbindungsstellen für eine Abdichtung der Medien untereinander sorgen.

Die Figuren 6 und 7, 9 und 10 sowie 11 und 12 zeigen jeweils zueinanderpassende Kombinationen von Einsatzteilen und Drehkupplungen, wobei diesen Kombinationen gemeinsam ist, daß die Einsatzteile hinsichtlich der äußeren Konturen stets der Hülse 4 des Handstückes 1 angepaßt, dagegen die Innenkonturen und die Medienübergabestellen unterschiedlich und den jeweiligen Zapfen der Drehkupplungen angepaßt sind. Demgemäß kann in das Handstück 1 anstelle des beschriebenen Einsatzteiles 7 auch das in Figur 9 gezeigte Einsatzteil 30 eingesetzt werden, welches zu der in Figur 10 dargestellten Drehkupplung 31 passend ist. Im Vergleich mit Figur 7 ist erkennbar, daß die Drehkupplung 31 hinsichtlich der Zapfenausbildung und auch hinsichtlich der Anbietung der Medien (TL, SL, SW und RL) konstruktiv anders gestaltet ist als die Drehkupplung nach Figur 7. Ein weiterer Unterschied ist, daß bei der Drehkupplung 31 elektrische Kontakte in Form von am Zapfenumfang angeordneten Schleifringen (SR) 32 vorhanden sind, die mit entsprechenden federnden Kontaktelementen (SK) 33 im Einsatzteil 30 zusammenwirken. Die Kontaktelemente 33 sind über elektrische Leitungen 34 mit einer Fassung für eine Lampe 35 verbunden. Obgleich die Medien RL, SL, TL und SW an anderen Stellen als bei der Ausführung in Figur 7 angeboten und im Einsatzteil an anderer Stelle von letzterem übernommen werden, so werden die Medien jedoch stirnseitig des Einsatzteiles 30 an gleicher Stelle abgegeben, wie bei dem Einsatzteil 7 nach Figur 6; d.h. das Lochbild für die Abgabe der Medien TL, SL und SW ist gleich dem Lochbild bei der Ausführungsform nach Figur 6.

Im Gegensatz zu der zuvor beschriebenen Ausführungsform, bei der die Lampe als lichterzeugendes Element stirnseitig des Zapfens der Drehkupplung 11 angeordnet ist, ist bei der zweiten Ausführungsform die Lampe 35 Bestandteil des Einsatzteiles 30; sie erhält die erforderliche Spannung über eine Kontaktanordnung 32, 33 in Form von Schleifringen (SR) und federnden Kontakten (SK).

Nachdem das Einsatzteil 30 in seinen äußeren Abmessungen (Läng/Durchmesser) identisch ist mit der Ausführung nach Figur 6 kann es alternativ zum Einsatzteil 7 in das Handstück eingesetzt werden.

Die Figuren 11 und 12 zeigen eine dritte Kombination einer Drehkupplung und eines Einsatzteiles. Aus der Darstellung geht hervor, daß die Drehkupplung 36 konstruktiv anders ausgebildet und die Medien TL, SL, SR, SW, RL auch an anderen Stellen anbietet als die zuvor beschriebenen Ausführungsformen. Demgemäß ist auch das Einsatzteil 37 so ausgebildet, daß die Leitungen und Kanäle die Medien zwar an anderen Stellen übernehmen, jedoch, da gleiches Lochbild für die Abgabe der Medien vorhanden ist, stirnseitig an der gleichen Stelle, wie bei den zuvor beschriebenen Ausführungen, wieder abgeben, so daß, wenn das Einsatzteil 37 in das Handstück 1 eingeführt wird, die Medien TL, SW und SL über die Kanäle 22 bis 24 in der zuvor erläuterten Weise zum Handstück geführt werden können. Nachdem bei der Ausführungsform nach Figur 12 Schleifringe (RL) nicht am Umfang des Zapfens, wie bei der Ausführung nach Figur 10, sondern an einer stirnseitigen Fläche 38 vorgesehen sind, enthält das Einsatzteil 37 im Durchmesser versetzt angeordnete federnde Kontaktstifte (SK) 39, die über Leitungen 40 mit einer wiederum im Zentrum des Einsatzteiles 37 angeordneten Lampe 41 verbunden sind.

Aus der Darstellung ist erkennbar, daß, je nachdem, auf welche Drehkupplung (11, 31 oder 36) das Handstück 1 aufgesetzt werden soll, ein der Drehkupplung angepaßtes Einsatzteil (7, 30, 37) einzusetzen ist. Das Handstück kann also, ohne daß es konstruktiv geändert zu werden braucht, allein durch Auswechseln des Einsatzteiles auf die Benutzung einer anderen Drehkupplung umgerüstet werden.

In den vorbeschriebenen Ausführungsformen ist davon ausgegangen worden, daß die Drehkupplungen jeweils gleiche Halte- und Rastmittel (Teile 5 und 6) zur axialen Fixierung des Handstückes im gekuppelten Zustand aufweisen. Sollte dies konstruktiv nicht möglich oder gegeben sein, so ist es vorteilhaft, in das jeweilige Einsatzteil gleichsam auch die Rastmittel zur Halterung des Handstückes an der Drehkupplung vorzusehen. Eine solche Ausführungsform ist in den Figuren 14 und 15 dargestellt. Diese Ausführungsform stellt praktisch eine Modifikation der in den Figuren 6 und 7 gezeigten Teile dar, und zwar insoweit, daß der Ringbund 42 zur Rastung der Drehkupplung 43 im Durchmesser kleiner ist als bei der in Figur 7 gezeigten Ausführungsform, wodurch sich die Drehkupplung nicht direkt an der Handstückhülse 4 rasten lassen würde. Um für einen solchen Fall eine Rastung zu ermöglichen, wird vorgeschlagen, daß das modifizierte Einsatzteil 44 die Mittel zur Rastung, z.B. in Form einer an sich bekannten Kugelrastung 45, enthält, wie dies in Figur 14 vereinfacht dargestellt ist. Das Handstück ist bei dieser Version demnach nicht durch bezüglich der konstruktiven Gestaltung der Drehkupplung, soweit sie die Medienführung betrifft, unabhängig, sondern auch bezüglich der Rastung des Handstückes an der Drehkupplung. Das Handstück ist demnach bei dieser Version noch neutraler und unabhängiger von der verwendeten Drehkupplung.

## Patentansprüche

1. Zahnärztliche Handstückanordnung, enthaltendeln Handstück (1) mit einem Kopfteil (2) und einer daran befestigten Griffhülse (4) und mehrere, unterschiedlich gestaltete und, verschiedene Versorgungsmedien, wie Luft, Wasser, elektrischen Strom und Licht liefernde und diese Versorgungsmedien an unterschiedlichen Stellen anbietende Drehkupplungen, (11, 31, 36, 43) welche fest oder lösbar mit einem Versorgungsschlauch (13) verbindbar sind, wobei für den beliebigen Anschluß des Handstückes (1) an eine der Drehkupplungen (11, 31, 36, 43) dem Handstück (1) mehrere Einsatzteile (7, 30, 37, 44) zugeordnet sind, die alternativ in einem an dem dem Kopfteil (2) abgewandten Ende des Handstückes angeordneten, hülsenförmigen Endabschnitt der Griffhülse(4) gehaltert werden können, wobei die Einsatzteile den Drehkupplungen zugewandt hinsichtlich der Anschlußmaße und der Medienübergabe verschieden und auf die unterschiedlichen Drehkupplungen abgestimmt sind, **dadurch gekennzeichnet,** daß die Einsatzteile (7, 30, 37, 44) dem Handstück zugewandt hinsichtlich der Außenabmessungen und der Medienübergabe gleich und auf das Handstück abgestimmt sind.

2. Handstückanordnung nach Anspruch 1, **dadurch gekennzeichnet**, daß der Endabschnitt des Handstückes (1) eine nur den äußeren Hüllmantel bildende dünne Hülse (4) bildet und sämtliche medienführenden Leitungen (19 bis 21) im Einsatzteil (7) angeordnet sind.

3. Handstückanordnung nach Anspruch 2, **dadurch gekennzeichnet**, daß die Medienübergabe vom Einsatzteil (7) zum Handstück (1) stirnseitig des Einsatzteils vorgesehen ist und sämtliche Einsatzteile (7, 30, 37, 44) an dieser Stirnseite gleiches Anschlußbild für die Medienübergabe aufweisen.

4. Handstückanordnung nach Anspruch 3, **dadurch gekennzeichnet**, daß das eine Einsatzteil (30, 37) an seinem dem Kopfteil (2) zugewandten Ende eine zentrisch angeordnete Lampe (35, 41) enthält, deren elektrische Kontakte mit einer am Kopfteil abgewandten Ende des Einsatzteiles angeordneten Kontaktanordnung (33, 39) verbunden sind, die mit an der zugehörigen Drehkupplung (31, 36) vorgesehenen Schleifringkontakten (32, 38) korrespondieren.

5. Handstückanordnung nach einem der Ansprüche 1 bis 4, **dadurch gekennzeichnet**, daß das Handstück (1) einen Lichtleiter (25) enthält, der dem Einsatzteil (7, 30, 37, 44) zugewandt zentrisch endet und dort im gekuppelten Zustand von Handstück und Drehkupplung einer in der Drehkupplung (11) oder im Einsatzteil (30, 37) angeordneten Lampe (17, 35, 41) korrespondierend gegenübersteht.

6. Handstückanordnung nach Anspruch 5, **dadurch gekennzeichnet**, daß zur Fixierung des dem Kopfteil (2) abgewandten Endes des Lichtleiters (25) eine Halterung (26) vorgesehen ist, welche zusammen mit dem hülsenförmigen Endabschnitt (4) des Handstückes (1) vorzugsweise im Querschnitt segmentförmige Durchströmöffnungen (27) zur Führung von Rückluft (RL) bildet.

7. Handstückanordnung nach Anspruch 6, **dadurch gekennzeichnet**, daß die Einsatzteile (7, 30, 37, 44) dazu passende Durchströmkanäle (28) für die Rückluft (RL) aufweisen.

8. Handstückanordnung nach einem der Ansprüche 1 bis 7, **dadurch gekennzeichnet**, daß die Einsatzteile (7, 30, 37, 44) rotationssymmetrische Innenkonturen aufweisen.

## Claims

1. Dental handpiece arrangement, comprising a handpiece (1) with a head part (2) and a grip sleeve (4) fastened thereto, and several differently formed rotatable couplings (11,31,36,43) delivering various supply media, such as air, water, electrical current and light and providing these supply media at different points, the rotatable couplings being capable of being connected fixedly or releasably to a supply tube (13), whereby a plurality of insert parts (7, 30, 37, 44) are associated with the handpiece (1) for any connection of the handpiece (1) to one of the rotatable couplings (11,31,36,43), which insert parts can be supported alternatively in a sleeve-shaped end section of the grip sleeve (4) arranged on the end of the handpiece which is directed away from the head part (2), whereby the insert parts facing the rotatable couplings are different with regard to the companion dimensions and the delivery of the media and are coordinated with the different rotatable couplings, characterized in that the insert parts (7,30,37,44) facing the handpiece are the same with regard to the outer dimensions and the delivery of the media and are matched with the handpiece.

2. Handpiece arrangement according to claim 1, characterized in that the end section of the handpiece (1) forms a thin sleeve (4) forming only the outer cover casing and all the media-supplying lines (19 to 21) are arranged in the insert part (7).

3. Handpiece arrangement according to claim 2, characterized in that the delivery of the media from the insert part (7) to the handpiece (1) is provided on the face of the insert part and all the insert parts (7, 30, 37, 44) at this face have the same attachment design for the delivery of the media.

4. Handpiece arrangement according to claim 3, characterized in that the one insert part (30, 37) at its end facing the head part (2) contains a centrally arranged lamp (35, 41), the electrical contacts of which are connected to a contact arrangement (33, 39) arranged at the end of the insert part which is directed away from the head part, the contact arrangements corresponding with slip-ring contacts (32, 38) provided at the matching rotatable coupling (31, 36).

5. Handpiece arrangement according to one of claims 1 to 4, characterized in that the handpiece (1) contains a photoconductor (25), which ends so that it faces the insert part (7, 30, 37, 44) centrally and when handpiece and rotatable coupling are coupled is correspondingly opposite a lamp (17, 35, 41) arranged in the rotatable coupling (11) or in the insert part (30, 37).

6. Handpiece arrangement according to claim 5, characterized in that to fix the end of the photoconductor (25) which is directed away from the head part (2) a holder (26) is provided, which forms together with the sleeve-shaped end section (4) of the handpiece (1) through-flow openings (27), which are preferably segment-shaped in cross-section, for the conduction of return air (RL).

7. Handpiece arrangement according to claim 6, characterized in that the insert parts (7, 30, 37, 44) have suitable through-flow ducts (28) for the return air (RL).

8. Handpiece arrangement according to one of claims 1 to 7, characterized in that the insert parts (7, 30, 37, 44) have rotationally symmetrical inner contours.

## Revendications

1. Dispositif à pièce à main de dentisterie comportant une partie de tête (2) et une douille de préhension (4) et plusieurs accouplements rotatifs agencés différemment, qui fournissent différents milieux d'alimentation tels que l'air, l'eau, le courant électrique et la lumière, et offrent ces milieux d'alimentation en différents emplacements et peuvent être raccordés de façon fixe ou amovible à un tuyau d'alimentation, plusieurs pièces d'insertion (7,30,37,40), qui peuvent être fixées ou non dans un tronçon terminal en forme de douille de la douille (4) de préhension disposée sur l'extrémité de la pièce à main, tournée à l'opposé de la partie de tête (2), étant associées à la pièce à main (1), les pièces d'insertion étant différentes, du côté tourné vers l'accouplement rotatif, pour ce qui concerne les cotes de raccordement et le transfert des milieux et étant réglées sur les différents accouplements rotatifs, caractérisé en ce que les pièces d'insertion (7,30,37,44) sont, du côté tourné vers la pièce à main, identiques pour ce qui concerne les dimensions extérieures et le transfert des milieux et adaptées aux accouplements rotatifs différents.

2. Dispositif à main suivant la revendication 1, caractérisée par le fait que la section terminale de la pièce à main (1) forme une douille mince (4) constituant uniquement l'enveloppe extérieure et que tous les conducteurs et conduits (19 à 21) véhiculant les milieux sont disposés dans la pièce d'insertion (7) .

3. Dispositif à main suivant la revendication 2, caractérisée par le fait que le transfert des milieux de la pièce d'insertion (7) à la pièce à main (1) est réalisé sur le côté frontal de la pièce d'insertion et que toutes les pièces d'insertion (7,30,37,44) possèdent, sur cette face frontale, la même configuration de raccordement pour le transfert des milieux,

4. Dispositif à main suivant la revendication 3, caractérisée par le fait qu'une pièce d'insertion (30,37) comporte, sur son extrémité tournée vers la partie de tête (2), une lampe centrée (35,41), dont les contacts électriques sont raccordés à un dispositif de contact (33,39) disposé sur l'extrémité, tournée à l'opposé de la partie de tête, de la pièce d'insertion, lesdits contacts correspondant aux contacts (32,38) de bague coulissante, qui sont prévus sur l'accouplement rotatif associé (31,36).

5. Dispositif à main suivant l'une des revendications 1 à 4, caractérisée par le fait que la pièce à main (1) comporte un guide de lumière (25), qui se termine en position centrée du côté tourné vers la pièce d'insertion (7,30,37,44) et qui, en cet endroit, est disposé, de façon correspondante, en vis-à-vis d'une lampe (17,35 à 41) située dans l'accouplement rotatif (11) ou dans la partie d'insertion (30,37), lorsque a pièce à main et l'accouplement rotatif sont accouplés.

6. Dispositif à main suivant la revendication 5, caractérisée par le fait que pour la fixation de l'extrémité, tournée à l'opposé de la partie de tête (2), du guide de lumière (25), il est prévu un support (26), qui forme, avec la section terminale en forme de douille (4) de la pièce à main (1), des ouvertures de passage (27) de préférence en forme de segments en coupe transversale pour le guidage de l'air de renvoi (RL).

7. Dispositif à main suivant la revendication 6, caractérisée par le fait que les pièces d'insertion (7,30,37,44) comportent des canaux de passage (28) adaptés pour la circulation de l'air de renvoi (RL).

8. Dispositif à main suivant l'une des revendications 1 à 7, caractérisée par le fait que les pièces d'insertion (7,30,37,44) possèdent des contours intérieurs à symétrie de révolution.
